# EUROPEAN PATENT APPLICATION

(11) **EP 2 353 594 A1**
(43) Date of publication of application: **10.08.2011**
(21) Application number: 10384001.3
(22) Date of filing: 09.02.2010
(51) Int. Cl.: A61K 31/135, A61K 31/415, A61P 25/00, A61P 25/02, A61P 25/04, A61P 29/00

(54) **Compositions comprising tramadol and the co-crystal of celecoxib and L-proline in the treatment of pain**

(71) Applicant: Laboratorios del. Dr. Esteve, S.A., 08041 Barcelona (ES)
(72) Inventor: Plata Salaman, Carlos Ramon, 08950 Esplugues de Llobregat (Barcelona) (ES)
(74) Representative: Peters, Hajo

(57) **Abstract**

The present invention relates to a compound combination of tramadol and a co-crystal of celecoxib ― L-proline or a pharmaceutical composition comprising it and their uses as medicaments, more particularly for the treatment of pain.

## Description

The present invention relates to a compound combination of tramadol and a co-crystal of celecoxib - L-proline or a pharmaceutical composition comprising it and their uses as medicaments, more particularly for the treatment of pain.

Pain is a complex response that has been functionally categorized into sensory, autonomic, motor, and affective components. The sensory aspect includes information about stimulus location and intensity while the adaptive component may be considered to be the activation of endogenous pain modulation and motor planning for escape responses. The affective component appears to include evaluation of pain unpleasantness and stimulus threat as well as negative emotions triggered by memory and context of the painful stimulus.

In general, pain conditions can be divided into chronic and acute. Chronic pain includes neuropathic pain and chronic inflammatory pain, for example arthritis, or pain of unknown origin, as fibromyalgia. Acute pain usually follows non-neural tissue injury, for example tissue damage from surgery or inflammation, or migraine.

The object of the present invention is to provide a pharmaceutical composition means for the treatment of pain by choosing a highly suitable combination of active compounds.

Clinical improvements/advantages desirable to the new pharmacological means would include any single one of the following or even more preferable more than one of the following advantages below:
● being most effective through different analgesic mechanisms providing more effective pain relief for a broader spectrum of pain;
● providing a new more effective method for treating acute or severe to moderate pain, especially pain with an inflammatory component;
● reducing adverse drug reactions (side effects) while having an at least additive effect of the active compounds of the highly suitable combination;
● reducing adverse drug reactions with a better safety profile at higher doses;
● allowing a reduction of dose while still delivering the desired activity using less of each ingredient and, therefore, reducing the side effects associated with each active principle; or
● increasing the level of efficacy or reaching at lest an efficacy similar to the one achievable by each active substance used alone at higher doses associated with a better safety profile.

This objective was achieved by providing a new compound combination of tramadol and co-crystal celecoxib ― L-proline or a pharmaceutical composition comprising it. Such a pharmaceutical composition seems to be very effective in the treatment of pain, especially for the treatment of acute or severe to moderate pain, especially in pain with an inflammatory component.

Therefore, the main object of the invention is a compound combination of tramadol as a free base or as a pharmaceutically acceptable salt or hydrate thereof and of co-crystal of celecoxib with L-proline or a pharmaceutical composition comprising it.

There are many drugs that are known to be useful in the treatment or management of pain. Opioids are frequently used as analgesics in pain. Derivatives of morphine are indicated for the treatment of moderate to acute pain in human. The analgesic effect is obtained through their action on morphinic receptors, preferably the µ-receptors. Among these derivatives of morphine, morphine, codeine, pethidine, dextropropoxyphenemethadone, lenefopan may be mentioned.

One of the morphinic derivatives that have shown very good results when orally administrated, and which is well marketed, is Tramadol, also available as a physiologically acceptable salt, particularly as a chlorohydrate. Tramadol is a central acting analgesic drug that exerts its effects by activating opioid receptors and enhancing neuronal monoamine synaptic concentration. Tramadol, whose chemical name is 2-(dimethylaminomethyl)-1-(3-methoxyphenyl)cyclohexanol, has the following formula :

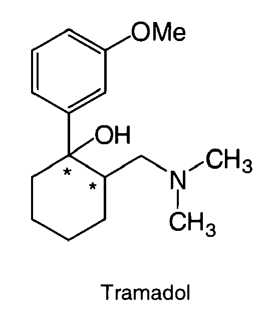

This structure shows two different chiral centers and thus may exist in two different diastereoisomers among which the tramadol is the cis-diastereisomer: (1*R*, 2*R*), or (1*S*, 2*S*). Accordingly, of tramadol there is a racemic form (called (rac)-tramadol) and two enantiomers (1*R,* 2*R*), or (1*S*, 2*S*)-tramadol. From the art it appears that this compound is neither fully opioid-like, nor non-opioid-like. Some studies have demonstrate that tramadol is an opioid agonist, whereas clinical experience indicates that it lacks many of the typical side effects of opioids agonist, for example respiratory depression, constipation or tolerance.

Due to their drawbacks, opioids as analgesics to treat pain cannot always be given repeatedly or at higher doses. For reviewing side effects of opioids one can mention J. Jaffe in "Goodman and Gilman's, The Pharmacological Basis of Therapeutics", 8th edition; Gilman et al.; Pergamon Press, New York, 1990, Chapter 22, pages 522-573.

Tramadol hydrochloride, which is often used orally, displays a highly bitter taste, which makes the drugs often difficult to swallow and lowers patient compliance. Also, as stated before, the drawbacks associated with opioids - their side effects - are limiting their use, so that they have to be given at lower doses and often less frequently as their use as analgesics to treat pain would normally require.

Consequently it has been proposed to combine opioids with other drugs that are not opioid analgesic agents, in order to lower the amount of opioids needed to produce an equivalent degree of analgesia. Among these combinations, the association of tramadol with nonsteroidal anti-inflammatory drugs (NSAIDs) has been reported to be of particular interest (EP-0 546 676). US 5,516,803 patent (family of EP-0 546 676) discloses combination of tramadol with nonsteroidal antiinflammatory drugs (NSAIDs), specifically ibuprofen and discloses that the combination of tramadol.HCl with non-steroidal anti-inflammatories, such as for example ibuprofen, in a composition ratio of 1:1 to 1:200 produces a synergistically enhanced analgesic action and reduces the undesired accompanying symptoms

US 6,558,701 patent discloses combination of tramadol with diclofenac and "for the treatment of moderate to severe pain, the World Health Organization (WHO) recommends combining opioid analgesics with non-steroidal analgesics in order to produce a more effective pain relief and possibly reduce amounts of analgesic which are necessary to administer".

One commonly known group of non-opioid analgesic agents are the well established COX-inhibitors. COX-inhibitors, as their name implies, are effective in relieving pain via inhibiting the enzymes cyclooxygenase (COX) I (constitutive) and/or II (inducible) involved in the production of prostaglandins. COX-II is the isoform of the enzyme that has been shown to be induced by pro-inflammatory stimuli and has been postulated to be primarily responsible for the synthesis of prostanoid mediators of pain, inflammation, and fever. COX-II is also involved in ovulation, implantation and closure of the ductus arteriosus, regulation of renal function, and central nervous system functions (fever induction, pain perception and cognitive function). It may also play a role in ulcer healing.

COX-inhibitors include non-steroidal anti-inflammatory drugs (NSAIDs) with acetylsalicylic acid known under its trademark Aspirin - despite being more than 100 years old - being an outstandingly used pharmaceutical. Besides Aspirin other COX-inhibitors whose use generally is also centred on anti-inflammatory action like ibuprofen, naproxen or diclofenac are among the worldwide most frequently applied pharmaceutical compounds.

One well-known NSAID is the marketed drug celecoxib, whose chemical name is 4-[5-(4-methylphenyl)-3-(trifluoromethyl)-pyrazol-1-yl]-benzenesulfonamide. Celecoxib is an antiinflammatory and pain killer drug and it is one of the most used treatments for chronic musculo-skeletal inflammatory illnesses. It has an empirical formula of C₁₇H₁₄F₃N₃O₂S.

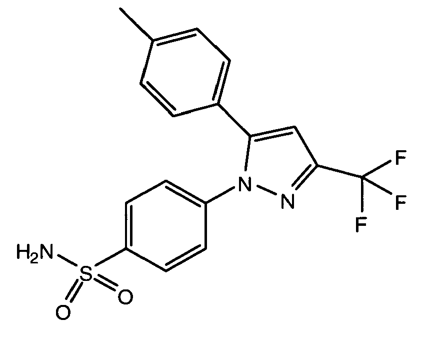

Celecoxib is an oral, highly selective cyclooxygenase-2 (COX-2) inhibitor, and it is indicated for the treatment of symptomatic relief in the treatment of osteoarthritis, rheumatoid arthritis and ankylosing spondylitis (Goldenberg MM. Celecoxib, a selective cyclooxygenase-2 inhibitor for the treatment of rheumatoid arthritis and osteoarthritis. Clin Ther. 1999, 21, 1497-513).This high selectivity allows celecoxib and other COX-2 inhibitors to reduce inflammation (and pain) while minimizing gastrointestinal adverse drug reactions (e.g. stomach ulcers) that are common with non-selective NSAIDs.

WO00/51685 describes pharmaceutical composition comprising on one hand a tramadol material selected from:
(+)- and (-)-tramadol, racemic tramadol, the N-oxide of tramadol and O-desmethyltramadol (both of them as isolated stereoisomers or mixtures thereof including their racemates) either as free base or as a salt, solvate or polymorph;
and on the other hand a selective COX-2 inhibitor with celecoxib being listed among the COX-2 inhibitor drugs. The focus of the application rests in the exemplified combination of tramadol and JT-522.

The advantage of COX-inhibitors in general, i.e. COX-I and COX-II inhibitors, is that they do not produce tolerance or physical dependence and are also not associated with abuse or addiction. However, for a number of COX-inhibitors a low solubility in water exists. This is especially true for the very popular and widely used and distributed members of the group of COX-inhibitors, naproxen, diclofenac and ibuprofen, whose poor solubility is a published fact, that has lead to considerably efforts for improvement by using solution enhancers etc. in their formulation.

The COX-11 inhibitor celecoxib is also well-known to be only slightly soluble limiting inter alia its use in pharmaceutical formulations and its bioavailability. For instance, the marketed formulation CelebrexO, that is administered orally, contains celecoxib in its crystalline form and has an absolute bioavailability ranging from about 20-40% in dogs attributed to a reduced absorption (Susan K. Paulson, Margaret B. Vaughn, Susan M. Jessen, Yvette Lawal, Christopher J. Gresk, Bo Yan, Timothy J. Maziasz, Chyung S. Cook. Aziz Karim, 2001, "Pharmacokinetics of Celecoxib after Oral Administration in Dogs and Humans: Effect of Food Site of Absorption", J. Pharmacol.& Experim. Therapeutics, 297(2), 638-645). This poor bioavailability limits the distribution and target organ delivery of celecoxib, and hence, the efficacy of this drug.

Celecoxib is weakly acidic with a pKa in water of 11.1 (http://www.medsafe.govt.nz/profs/Datasheet/c/Celebrexcap.htm) leading to the non-ionized form at all physiological pH ranges. Therefore, celecoxib is a high permeable drug exhibiting a very low water solubility of 7µg/ml (Neelam Seedher, Somm Bhatia, 2003 "Solubility enhancement of Cox-2 Inhibitors Using various Solvent Systems", AAPS Pharm. Sci. Tech., 4(3), 1-9). These physico-chemical properties make celecoxib to be considered as class II in the Biopharmaceutical Classification System (BCS) (Mehran Yazdanian, Katherine Briggs, Corinne Jankovskty, Amale Hawi, 2004, "The "High solubility" Definition of the Current FDA Guidance on Biopharmaceutical Classification System May Be Too Strict for Acidic Drugs", Pharm. Res., 2004, 21 (2), 293-9). Consequently the extent of celecoxib oral absorption appears to be limited by its bad solubility and slow dissolution rate, which leads to an inadequate dissolution in gastrointestinal fluids (Susan K. Paulson, Margaret B. Vaughn, Susan M. Jessen, Yvette Lawal, Christopher J. Gresk, Bo Yan, Timothy J. Maziasz, Chyung S. Cook. Aziz Karim, "Pharmacokinetics of Celecoxib after Oral Administration in Dogs and Humans: Effect of Food Site of Absorption", J. Pharmacol.& Experim Therapeutics, 2001, 297(2), 638-645).

Thus, if having focussed on a compound combination like the one of tramadol and celecoxib as a solution to the basic underlying problem of providing pharmaceutical composition means for the treatment of pain, it became a further objective of the current invention to identify a composition of these active ingredients that cancel or ameliorate their drawbacks or other negative effects they have like improving the aqueous solubility and dissolution rate of celecoxib as well as the retard of precipitation of dissolved celecoxib or masking the bitter taste of tramadol or avoiding/ameliorating any of its opioid-like side-effects.

As said above it is considered very desirable if the compound combination comprising an opioid like tramadol and an NSAID like celecoxib in form of the co-crystal with L-proline according to the invention or its respective pharmaceutical composition would be having a level of efficacy similar to the one achievable by each active substance used alone, but:
➢ with a better safety profile at higher doses and/or
➢ - by showing a synergistic effect - allowing a reduction of dose while still delivering the desired activity using less of each ingredient and, therefore, reducing the side effects associated with each active principle; and/or
➢ providing a new more effective method for treating acute or severe to moderate pain, especially in pain with an inflammatory component; and/or
➢ enhancing patient compliance; and/or
➢ enhancing solubility of any of the active principles involved.

The compound combination or pharmaceutical composition according to the invention shows or in first impressions seems to show many advantages in its treatment potential.

Especially and at first impression, the compound combination or pharmaceutical composition according to the invention seems to be capable of fulfilling also one or even some of the desirable clinical advantages listed above (especially if compared to any of the active principles alone), like
● providing more effective pain relief for a broader spectrum of pain,
● reducing adverse drug reactions providing a better safety profile at higher doses,
● reducing side effects with an additive effect of the compounds in the combination,
● increasing the level of efficacy or reaching at least an efficacy similar to the one achievable by each active substance used alone at higher doses associated with a better safety profile, or
● allowing a reduction of dose with the desired activity reducing the side effects.

The applicant has now found that tramadol, especially the (rac)-tramadol hydrochloride salt, having the opioid activity, and co-crystal celecoxib - L-proline can be combined in one pharmaceutical composition achieving an additive effect, especially in the treatment of severe to moderate pain, especially in pain with an inflammatory element, with signs of reduced side effects.

Pharmaceutical compositions comprising a combination of tramadol and co-crystal celecoxib ― L-proline even seem to exhibit synergistic effects, thus, allowing for a highly efficient dose/weight relation of both active principles.

Already one part of the compound combination or pharmaceutical composition, the co-crystal celecoxib - L-proline, shows improved properties if compared to celecoxib alone and/or to a mixture of celecoxib and L-proline. The formulation of the association as a co-crystal is even easier with a solid to manipulate and shows enhanced stability and/or solubility. Another advantage is that the combination of one active principle like celecoxib with a co-crystal former like L-proline in one unique species allows for a better Pharmacokinetic/Pharmacodynamic (PKPD) which helps in the treatment of pain. Also the quite bitter taste related to tramadol seems to have been better masked.

In one embodiment in the combination according to the invention or in the pharmaceutical composition comprising it, the tramadol is (rac)-tramadol, (1*R*,1*R*)-tramadol or (1*S*,1*S*)-tramadol, preferably the tramadol is (rac)-tramadol.

In another embodiment in the combination according to the invention or in the pharmaceutical composition comprising it, tramadol is in form of a salt, preferably in form of a hydrochloride salt; most preferably wherein the tramadol is (rac)-tramadol hydrochloride salt.

As celecoxib is weakly acidic with a pKa of 11.1 its "neutral form" according to the invention is defined therefore as the form in which celecoxib is free (not in form of a salt) but is - depending on the pH - neutral or carrying a load.

In another embodiment in the combination according to the invention or in the pharmaceutical composition comprising it, the co-crystal comprises (or is formed from) celecoxib either in neutral form or as a physiologically salt and L-proline either as a free amino acid or as a physiologically salt, preferably wherein the co-crystal comprises (or is formed from) celecoxib in neutral form.

In another embodiment in the combination according to the invention or in the pharmaceutical composition comprising it, the molecular ratio between celecoxib and L-proline in the co-crystal is 1:2. Thereby, it is preferred if the endothermic sharp peak of the co-crystal corresponding to the melting point has an onset at 201.4°C, if the co-crystal shows a X-Ray powder diffraction pattern with peaks [2θ] at 5.29, 5.77, 7.28, 9.71, 10.60, 11.57, 12.39, 13.05, 13.56, 14.46, 15.11, 15.45, 15.83, 16.14, 16.96, 17.25, 17.67, 17.92, 18.08, 18.70, 19.37, 19.64, 19.85, 20.17, 20.43, 20.79, 21.17, 21.58, 21.85, 22.15, 22.30, 22.74, 23.12, 23.78, 24.04, 24.72, 24.87, 25.43, 26.57, 26.97, 27.66, 28.04, 28.37, 28.59, 29.11, 29.56, 29.78, 30.41, 30.60, 31.56, 32.21, 32.74, 33.13, 33.35, 33.75, 34.97, 35.80, 36.87, 37.48, 37.69, 38.08 and 38.87; or if the co-crystal has an orthorhombic unit cell with the following dimensions:
a = 9.61 Å,
b = 20.07 Å, and
c = 30.63 Å.

In a further preferred embodiment of the compound combination according to the present invention or pharmaceutical composition comprising it, tramadol and co-crystal celecoxib ― L-proline are present in a weight ratio based on a fraction of their respective ED₅₀ values which ratio may vary from about 1:1 to about 1:300 or, reversibly, from about 1:1 to about 300:1.

In a further preferred embodiment of the compound combination according to the present invention or pharmaceutical composition comprising it, tramadol and co-crystal celecoxib - L-proline are present in a weight ratio varying from about 1:1 to about 1:30 or, reversibly, from about 1:1 to about 30:1.

Another aspect of the invention relates to a process for the production of a combination according to the invention or of the pharmaceutical composition comprising it comprising the steps of:
(a) dissolving or suspending celecoxib and L-proline in a solvent or mixture of solvents,
(b) optionally mixing or stirring the solution or suspension,
(c) optionally before, during or after step (b) cooling or keeping the mixed solution/suspension to or at ambient temperature or below;
(d) optionally filtering-off and/or washing the resulting solid with a solvent, and
(e) drying the solid optionally at ambient temperature and/or vacuum; followed by
(f) mixing the resulting co-crystal with tramadol as a free base or as a pharmaceutically acceptable salt or hydrate thereof.

"Ambient temperature" is defined here as a temperature between 20 and 25°C, preferably being 20°C. Preferably the cooling in step (c) is done from ambient temperature to approximately 0-5°C.

The solvents usable in this process include water or organic solvents, preferably solvents selected from dichloromethane, methanol, acetone, isobutyl acetate, acetonitrile, ethyl acetate, 2-butanol, dimethylcarbonate, chlorobenzene, butylether, diisopropylether, dimethylformamide, ethanol, hexane, isopropanol, methyl ethyl ketone, methyl isobutyl ketone, methyl t-butyl ether, 3-pentanone, toluene and 1,1,1-trichloroethane, most preferably selected from dichloromethane, toluene, isobutyl acetate and methanol.

In one embodiment of the process according to the invention, the molecular ratio of celecoxib to L-proline in step a) is 1:2.

For completeness, disclosed herein is also a process for the production of a co-crystal to be used in the compound combination according to the present invention or the pharmaceutical composition comprising it, comprising the steps of:
(aa) dissolving or suspending celecoxib and L-proline in a solvent or mixture of solvents,
(bb) optionally mixing or stirring the solution or suspension,
(cc) optionally before, during or after step (b) cooling or keeping the mixed solution/suspension to or at ambient temperature or below;
(dd) optionally filtering-off and/or washing the resulting solid with a solvent, and
(ee) drying the solid optionally at ambient temperature and/or vacuum.

"Ambient temperature" is defined here as a temperature between 20 and 25°C, preferably being 20°C. Preferably the cooling in step (cc) is done from ambient temperature to approximately 0-5°C.

In one embodiment of this disclosed process for the production of a co-crystal, the molecular ratio of celecoxib to L-proline in step (aa) is 1:2.

The solvents usable in this disclosed process for the production of a co-crystal include water or organic solvents, preferably solvents selected from dichloromethane, methanol, acetone, isobutyl acetate, acetonitrile, ethyl acetate, 2-butanol, dimethylcarbonate, chlorobenzene, butylether, diisopropylether, dimethylformamide, ethanol, hexane, isopropanol, methyl ethyl ketone, methyl isobutyl ketone, methyl t-butyl ether, 3-pentanone, toluene and 1,1,1-trichloroethane, most preferably selected from dichloromethane, toluene, isobutyl acetate and methanol.

In another aspect of the invention, the invention relates to a pharmaceutical composition comprising a compound combination according to the invention.

A pharmaceutical composition according to this invention allows the use of a lower therapeutic dose of tramadol and celecoxib.

The association of two active elements in the same pharmaceutical compound, such as a co-crystal, exhibits several advantages transferred into the pharmaceutical composition according to the invention. Being linked, the elements often behave as a single chemical entity, thus facilitating the treatments, formulation, dosage etc. Another advantage is that the association of two active elements into one unique species seems to allow for a better Pharmacokinetic/Pharmacodynamic (PKPD) which helps in the treatment of pain.

In general in most embodiments in which a co-crystals of celecoxib and L-proline of the combination according to the invention or the combination itself is used (e.g. for the treatment of pain) the co-crystal or combination would be formulated into a convenient pharmaceutical formulation or a medicament. Accordingly, a desirable advantage of a pharmaceutical compound according to the present invention would show improved pharmaceutical properties and features, especially when compared to celecoxib alone, a mixture of celecoxib and L-proline and/or even a physical mixture of tramadol, celecoxib and L-proline. Thus, the pharmaceutical composition according to the invention should desirably show at least one, preferably more, of the following features:
● to have a very small particle size, e.g. from 300 µm or lower; or
● to be and/or remain essentially free of agglomerates; or
● to be less or not very hygroscopic; or
● to help in formulating controlled release or immediate release formulations; or
● to have a high chemical stability; or
if given to a patient
● to decrease the inter- and intra-subject variability in blood levels; or
● to show a good absorption rate (e.g. increases in plasma levels or AUC); or
● to show a high maximum plasma concentration (e.g. Cₘₐₓ); or
● to show decreased time to peak drug concentrations in plasma (tₘₐₓ); or
● to show changes in half life of the compound (t_{1/2}), in whichever direction this change is preferably directed.

In a further aspect the present invention relates to a pharmaceutical composition according to the present invention comprising an effective amount of tramadol in combination with co-crystal celecoxib - L-proline for treating acute to moderate pain, preferably with inflammation component. For example: treatment of osteoarthritis, rheumatoid arthritis, ankylosing spondylitis, sciatica (sciatic neuritis) or frozen shoulder.

In a preferred embodiment the pharmaceutical composition according to the invention is for the treatment of acute to moderate pain with inflammation component or for the treatment of pain, preferably acute pain, chronic pain, neuropathic pain, hyperalgesia, allodynia, cancer pain, diabetic neuropathy, diabetic peripheral neuropathy, fibromyalgia, osteoarthritis, rheumatoid arthritis, ankylosing spondylitis, sciatica, frozen shoulder or dysmenorrhea.

All active principles forming part of the pharmaceutical composition are well-known drugs used for a long time worldwide. Due to the therapeutic interest in tramadol in the treatment of pain symptoms and the well-known properties of celecoxib in this field of medical indication, a further object of the present invention is a medicament containing a pharmaceutical composition comprising tramadol and co-crystal of celecoxib with L-proline.

Thus, the invention also relates to a medicament comprising at least one combination according to the invention or a pharmaceutical composition comprising tramadol and co-crystal of celecoxib with L-proline according to the invention as described above and optionally one or more pharmaceutically acceptable excipients.

The medicament or pharmaceutical composition according to the present invention may be in any form suitable for the application to humans and/or animals, preferably humans including infants, children and adults and can be produced by standard procedures known to those skilled in the art. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986). The respective descriptions are hereby incorporated by reference and form part of the disclosure. The composition of the medicament may vary depending on the route of administration.

The medicament or pharmaceutical composition of the present invention may for example be administered parenterally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments or pharmaceutical compositions according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) and Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments or pharmaceutical compositions according to the present invention may also comprise an enteric coating, so that their dissolution is dependent on pH-value. Due to said coating the medicament may pass the stomach non-dissolved and the respective salts or their respective crystalline form is liberated in the intestinal tract. Preferably the enteric coating is soluble at a pH value of 5 to 7.5. Suitable materials and methods for the preparation are known from the prior art.

Typically, the medicaments or pharmaceutical compositions according to the present invention may contain 1-60 % by weight of the combination of tramadol and co-crystal celecoxib ― L-proline and 40-99 % by weight of one or more auxiliary substances (additives/excipients).

The medicaments or pharmaceutical compositions of the present invention may also be administered topically or via a suppository.

The daily dosage for humans and animals may vary depending on factors that have their basis in the respective species or other factors, such as age, sex, weight or degree of illness and so forth. The daily dosage for humans of the combination of tramadol and co-crystal celecoxib - L-proline according to the present invention preferably is in the range of 1 to 2000 mg, preferably 5 to 500 milligrams being administered during one or several intakes per day.

In a further aspect the present invention relates to the use of the compound combination of tramadol and co-crystal celecoxib ― L-proline according to the present invention or of the pharmaceutical composition comprising the combination of tramadol and co-crystal celecoxib ― L-proline according to the present invention for the treatment of pain, preferably acute pain, chronic pain, neuropathic pain, hyperalgesia, allodynia, cancer pain, diabetic neuropathy, diabetic peripheral neuropathy, fibromyalgia, osteoarthritis, rheumatoid arthritis, ankylosing spondylitis, sciatica, frozen shoulder or dysmenorrhea. The use might especially be drawn to the treatment of acute to moderate pain with an inflammatory component like e.g. osteoarthritis rheumatoid arthritis, ankylosing spondylitis, sciatica and frozen shoulder. Preferably this use is provided in the form of a medicament or a pharmaceutical composition according to the invention as described above.

In another aspect the present invention relates to the use of the combination of tramadol and co-crystal celecoxib - L-proline according to the present invention or of the pharmaceutical composition comprising the combination of tramadol and co-crystal celecoxib - L-proline according to the present invention in the production of a medicament for the treatment of pain, preferably acute pain, chronic pain, neuropathic pain, hyperalgesia, allodynia, cancer pain, diabetic neuropathy, diabetic peripheral neuropathy, fibromyalgia, osteoarthritis, rheumatoid arthritis, ankylosing spondylitis, sciatica, frozen shoulder or dysmenorrhea. The use might especially be drawn to the production of a medicament for the treatment of acute to moderate pain with an inflammatory component like e.g. osteoarthritis rheumatoid arthritis, ankylosing spondylitis, sciatica and frozen shoulder.

"Pain" is defined by the International Association for the Study of Pain (IASP) as "an unpleasant sensory and emotional experience associated with actual or potential tissue damage, or described in terms of such damage (IASP, Classification of chronic pain, 2nd Edition, IASP Press (2002), 210). Even though pain is always subjective its causes or syndromes can be classified.

"Sciatica" or "sciatic neuritis is defined herein as a set of symptoms including pain that derive from irritation of the sciatic nerve or its roots.

"Frozen shoulder" or "adhesive capsulitis" is defined herein as a symptom wherein the connective tissue surrounding the shoulder joint or the shoulder capsule itself is causing chronic pain, becoming inflamed and stiff.

"Ankylosing spondylitis" or "Morbus Bechterew" is a chronic, inflammatory arthritis and autoimmune disease. It mainly affects joints in the spine and the sacroilium in the pelvis, causing eventual fusion of the spine.

"Dysmenorrhea" (or dysmenorrhoea) is a medical condition characterized by severe uterine pain during menstruation.

One further aspect of the present invention relates to a method of treatment of pain, preferably acute pain, chronic pain, neuropathic pain, hyperalgesia, allodynia, cancer pain, diabetic neuropathy, diabetic peripheral neuropathy, fibromyalgia, osteoarthritis, rheumatoid arthritis, ankylosing spondylitis, sciatica, frozen shoulder or dysmenorrhea by providing to a patient in need thereof a sufficient amount of the combination of tramadol and co-crystal celecoxib - L-proline according to the present invention or of the pharmaceutical composition comprising the combination of tramadol and co-crystal celecoxib - L-proline according to the present invention. This method of treatment might especially be relevant for the treatment of acute to moderate pain with an inflammatory component like e.g. osteoarthritis rheumatoid arthritis, ankylosing spondylitis, sciatica and frozen shoulder. Preferably the combination or composition according to the invention is provided in physiologically suitable form like e.g. in the form of a medicament or a pharmaceutical composition according to the invention as described above.

The present invention is illustrated below with the help of the following examples. These illustrations are given solely by way of example and do not limit the invention.

### Brief description of the figures:

**Figure 1****:**
   Differential scanning calorimetry (DSC) analysis of the co-crystal celecoxib - L-proline (1:2).
**Figure 2****:**
   Thermogravimetric analysis (TGA) of the co-crystal celecoxib - L-proline (1:2).
**Figure 3****:**
   X-ray powder diffraction (XRPD) pattern of the co-crystal celecoxib - L-proline (1:2).
**Figure 4****:**
   Unit cell contents of the crystal structure of the co-crystal celecoxib - L-proline (1:2); obtained by single crystal X-ray diffraction analysis (SCXRD) showing four molecules of L-proline and two molecules of celecoxib (hydrogen atoms have been omitted for clarity; program used: Mercury 2.2).

### EXAMPLES

### General Remarks:

Generally all figures of crystal structures are depicted with hydrogen atoms being omitted for clarity. The program used was Mercury 2.2.
In all XRPD measurements the 2θ values were obtained using copper radiation (Cu_{Kα} 1.54060 Å).

### Example 1

### Preparation of a pharmaceutical combination comprising a compound combination of tramadol hydrochloride and co-crystal of celecoxib with L-proline and of pharmaceutical compositions comprising them

The preparation of pharmaceutical compositions comprising different ratios of compound combinations of a tramadol to co-crystal of celecoxib with L-proline are effected by preparing solutions having concentrations or suspensions expressed in mg of active drug per 10 mL of distilled water or in mg of active drug per suspension of 10 mL of 0.5% hydroxypropyl methylcellulose in distilled water. For example, 40 mg of a tramadol hydrochloride and 40 mg of co-crystal celecoxib - L-proline (1:2) are added to a 10 mL suspension of 0.5% hydroxypropyl methylcellulose in distilled water, yielding a 10 mL suspension of a 1:1 weight ratio between tramadol and co-crystal based on weight (40 mg:40 mg). The range of doses for each ratio tested is prepared separately in a similar manner. Accordingly, other ratios of pharmaceutical compositions comprising a combination of a tramadol hydrochloride and co-crystal celecoxib - L-proline may also be similarly prepared at various concentrations.

### Example 2:

### Preparation of a co-crystal of celecoxib and L-proline (1:2)

### Process A (preparation by slurrying):

To a 10 mL flask equipped with magnetic stirrer containing celecoxib (509 mg, 1.34 mmol) and L-proline (307 mg, 2.68 mmol, 2 eq), was added 4 mL dichloromethane. The resultant suspension was stirred overnight. The solid was filtered with a sintered funnel (porosity 3) and washed with 1 mL cold dichloromethane. Traces of solvent were removed *in vacuo* at room temperature affording co-crystal celecoxib - L-proline (1:2) as a white powder (768 mg, 94%).

### Process B (preparation by crystallization):

To an assay tube equipped with magnetic stirrer containing celecoxib (69 mg, 0.18 mmol) and L-proline (42 mg, 0.36 mmol, 2 eq), was added the minimum amount of methanol to obtain complete dissolution at 64°C (vol. methanol 0.35 mL). The solution was allowed to cool to ambient temperature without stirring. Crystallization took place during the following 7 days. The product was filtered with a sintered funnel (porosity 3) and traces of solvent were removed *in vacuo* at room temperature affording co-crystal celecoxib - L-proline (1:2) as transparent rosettes (81 mg, 73%).

### CHARACTERISATION OF THE CO-CRYSTAL:

The celecoxib - L-proline (1:2) co-crystal obtained according to the example was fully characterised by ¹H-NMR, FTIR, X-ray diffraction, Karl-Fisher analysis, DSC and TG and also single crystal X-ray diffraction analysis.

### ¹H NMR

Proton nuclear magnetic resonance analyses were recorded in methanol-d₄ in a Varian Mercury 400 spectrometer, equipped with a broadband probe ATB 1H/19F/X of 5 mm. Spectra were acquired dissolving 5-10 mg of sample in 0.6 mL of deuterated solvent.

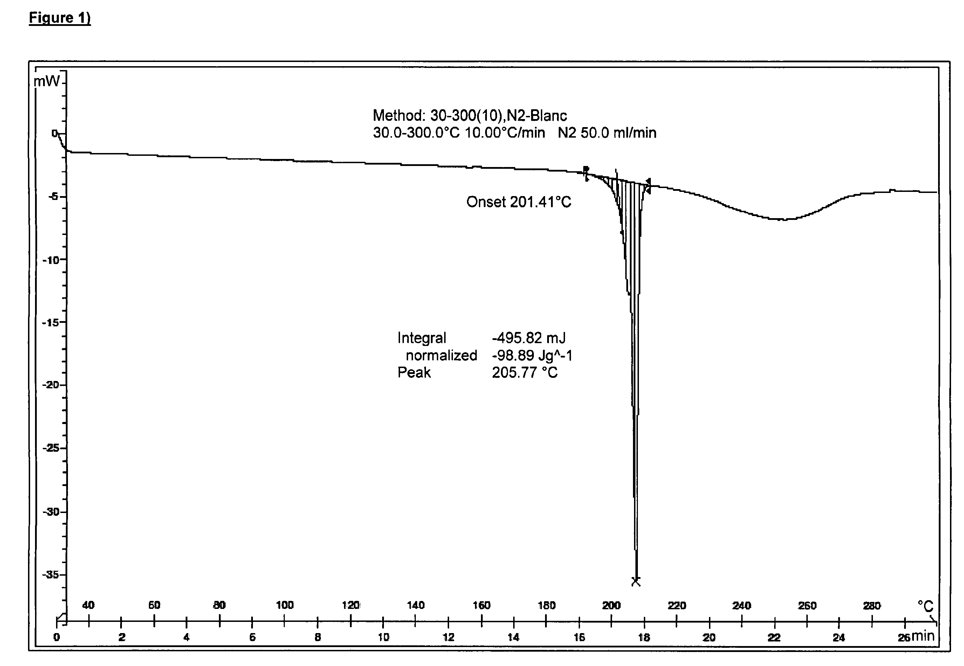

¹H NMR spectrum (see figure 1) in methanol-d₄ at 400 MHz shows peaks at 7.99-7.90 (m, 2H); 7.53-7.45 (m, 2H); 7.23-7.13 (m, 4H); 6.90 (s, 1H); 3.96 (dd, J = 6.2 Hz, J = 8.6 Hz, 2H); 3.38 (dt, J = 7.0 Hz, J = 11.7 Hz, 2H); 3.22 (dt, J = 7.4 Hz, J = 11.7 Hz, 2H); 2.35 (s, 3H); 2.35-2.23 (m, 2H); 2.16-2.05 (m, 2H) 2.03-1.90 (m, 4H) ppm.

### IR

FTIR spectra were recorded using a Thermo Nicolet Nexus 870 FT-IR, equipped with a beamsplitter KBr system, a 35 mW He-Ne laser as the excitation source and a DTGS KBr detector. The spectra were acquired in 32 scans at a resolution of 4 cm⁻¹.

The sample (KBr pellet) shows a Fourier Transform Infra Red spectrum with absorption bands at 3283.4 (m), 3079.5 (m), 2985.0 (m), 2680.0 (w), 1599.0 (m), 1500.1 (m), 1469.8 (s), 1449.2 (m), 1373.7 (s), 1345.4 (s), 1331.6 (s), 1267.3 (m), 1231.8 (s), 1172.0 (s), 1137.1 (s), 1111.9 (m), 1093.9 (m), 975.5 (m), 841.3 (s), 817.0 (s), 760.5 (m), 684.0 (m), 627.0 (s), 616.4 (s) 557.3 (m), 544.5 (m), 479.1 (m) cm⁻¹.

### DSC

Differential scanning calorimetry (DSC) analyses were recorded with a Mettler DSC822^{e}. A sample of 5.0140 mg was weighed into 40 µL aluminium crucible with a pinhole lid and was heated, under nitrogen (50 mL/min), at 10°C/min from 30 to 300°C.

The novel type of crystal of the present invention is characterized in that the endothermic sharp peak corresponding to the melting point has an onset at 201.4°C (fusion enthalpy - 98.89 J/g), measured by DSC analysis (10 °C/min) (see figure 1).

### TGA

Thermogravimetric analyses (TGA) were recorded in a thermogravimetric analyzer Mettler TGA/SDTA851^{e}. A sample of 4.5714 mg was weighed into a 70 µL alumina crucible with a pinhole lid and was heated at 10 °C/min from 30 to 300°C, under nitrogen (50 mL/min).

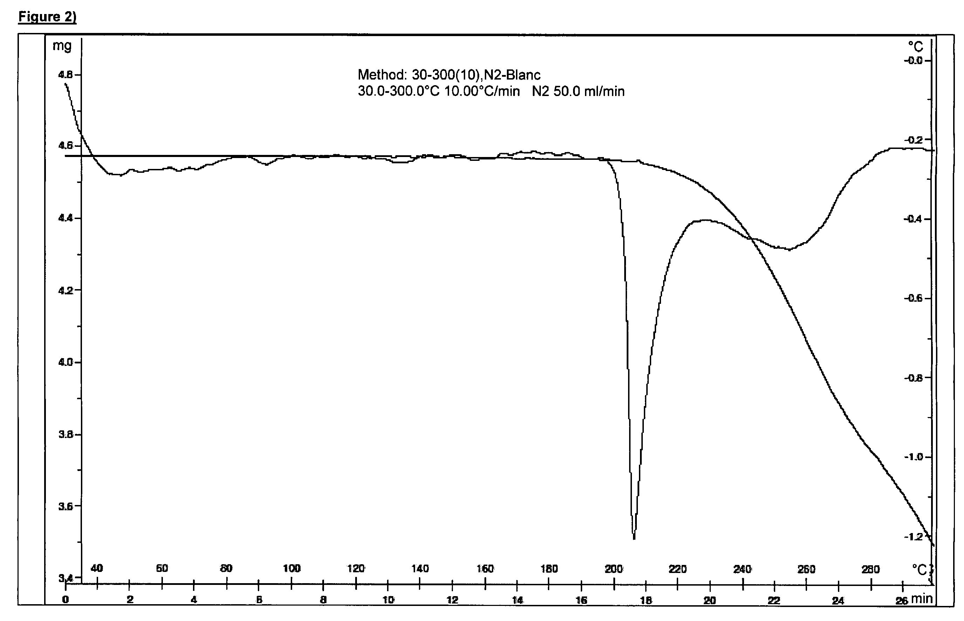

The TG analysis of the crystalline form according to the invention shows no weight loss between 30°C and its melting point (see figure 2).

### XRPD

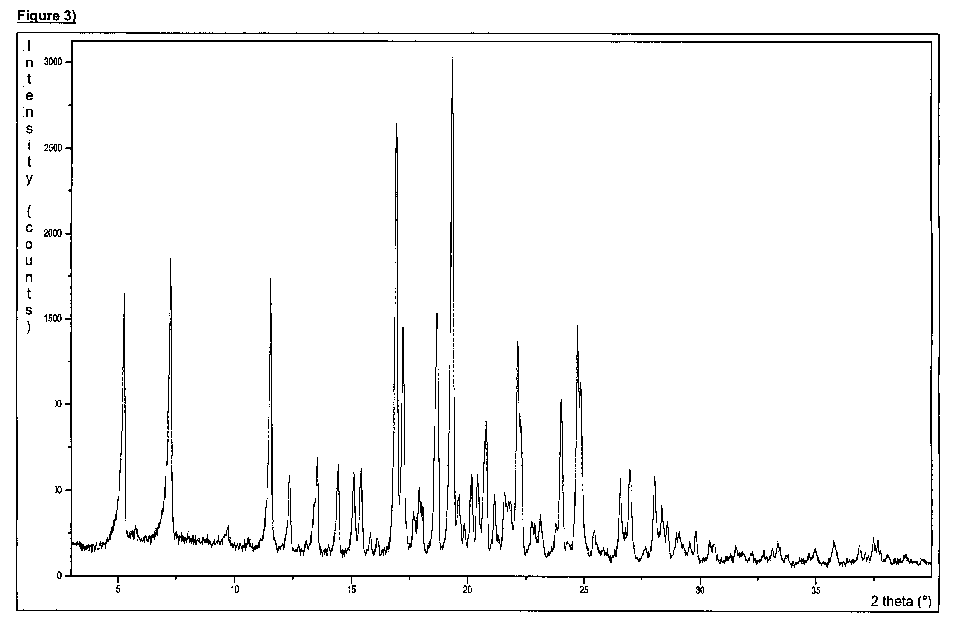

X-ray powder diffraction (XRPD) analysis was performed using a Philips X'Pert diffractometer with Cu K_{α} radiation in Bragg-Brentano geometry. The system is equipped with a monodimensional, real time multiple strip detector. The measurement parameters were as follows: the range of 20 was 3° to 40° at a scan rate of 8.8° per minute (see figure 3).

### List of selected peaks:

| **2θ (°)** | **d (Ä)** | **I (%)** |
|---|---|---|
| 5.29 | 16.72 | 54 |
| 5.77 | 15.32 | 2 |
| 7.28 | 12.14 | 59 |
| 9.71 | 9.11 | 4 |
| 10.60 | 8.35 | 1 |
| 11.57 | 7.65 | 58 |
| 12.39 | 7.15 | 15 |
| 13.05 | 6.79 | 1 |
| 13.56 | 6.53 | 19 |
| 14.46 | 6.13 | 19 |
| 15.11 | 5.86 | 17 |
| 15.45 | 5.74 | 19 |
| 15.83 | 5.60 | 4 |
| 16.14 | 5.49 | 3 |
| 16.96 | 5.23 | 94 |
| 17.25 | 5.14 | 48 |
| 17.67 | 5.02 | 7 |
| 17.92 | 4.95 | 13 |
| 18.08 | 4.91 | 8 |
| 18.70 | 4.74 | 48 |
| 19.37 | 4.58 | 100 |
| 19.64 | 4.52 | 10 |
| 19.85 | 4.47 | 4 |
| 20.17 | 4.40 | 15 |
| 20.43 | 4.35 | 15 |
| 20.79 | 4.27 | 27 |
| 21.17 | 4.20 | 10 |
| 21.58 | 4.12 | 11 |
| 21.85 | 4.07 | 10 |
| 22.15 | 4.01 | 47 |
| 22.30 | 3.99 | 26 |
| 22.74 | 3.91 | 6 |
| 23.12 | 3.85 | 9 |
| 23.78 | 3.74 | 6 |
| 24.04 | 3.70 | 34 |
| 24.72 | 3.60 | 51 |
| 24.87 | 3.58 | 37 |
| 25.43 | 3.50 | 5 |
| 26.57 | 3.35 | 17 |
| 26.97 | 3.31 | 19 |
| 27.66 | 3.23 | 2 |
| 28.04 | 3.18 | 17 |
| 28.37 | 3.15 | 11 |
| 28.59 | 3.12 | 7 |
| 29.11 | 3.07 | 5 |
| 29.56 | 3.02 | 3 |
| 29.78 | 3.00 | 5 |
| 30.41 | 2.94 | 3 |
| 30.60 | 2.92 | 3 |
| 31.56 | 2.83 | 3 |
| 32.21 | 2.78 | 1 |
| 32.74 | 2.74 | 2 |
| 33.13 | 2.70 | 3 |
| 33.35 | 2.69 | 4 |
| 33.75 | 2.66 | 1 |
| 34.97 | 2.57 | 3 |
| 35.80 | 2.51 | 4 |
| 36.87 | 2.44 | 3 |
| 37.48 | 2.40 | 5 |
| 37.69 | 2.39 | 4 |
| 38.08 | 2.36 | 1 |
| 38.87 | 2.32 | 1 |

### Single crystal X-ray diffraction

Crystal structure of co-crystal celecoxib - L-proline (1:2) has been determined from single crystal X-ray diffraction data. The colourless prismatic crystal used (0.52 x 0.33 x 0.33 mm) was obtained from the crystallization of a solution in toluene and methanol of equimolar amounts of celecoxib and L-proline.

Analysis was performed at room temperature using a Bruker Smart Apex diffractometer with graphite monochromated Mo K_{α} radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0).

The structure was solved with direct methods and least-squares refinement of Fₒ² against all measured intensities was carried out (program used: SHELXTL-NT 6.1). All non-hydrogen atoms were refined with anisotropic displacement parameters. Fluorine atoms and some carbons in the structure were found to be affected by disorder.

### Relevant structural data:

| Crystal system | Orthorhombic |
|---|---|
| Space group | *P*2₁2₁2₁ |
| a (Å) | 9.6086(4) |
| b (Å) | 20.0675(7) |
| c (Å) | 30.6272(12) |
| Volume (Å³ | 5905.6(4) |
| Z | 8 |
| D calc. (Mg/m³) | 1.38 |
| N. of refl. | 14575 |
| Refl. with I > 2σ(I) | 10837 |
| R (I>2σ(I)) | 0.0614 |

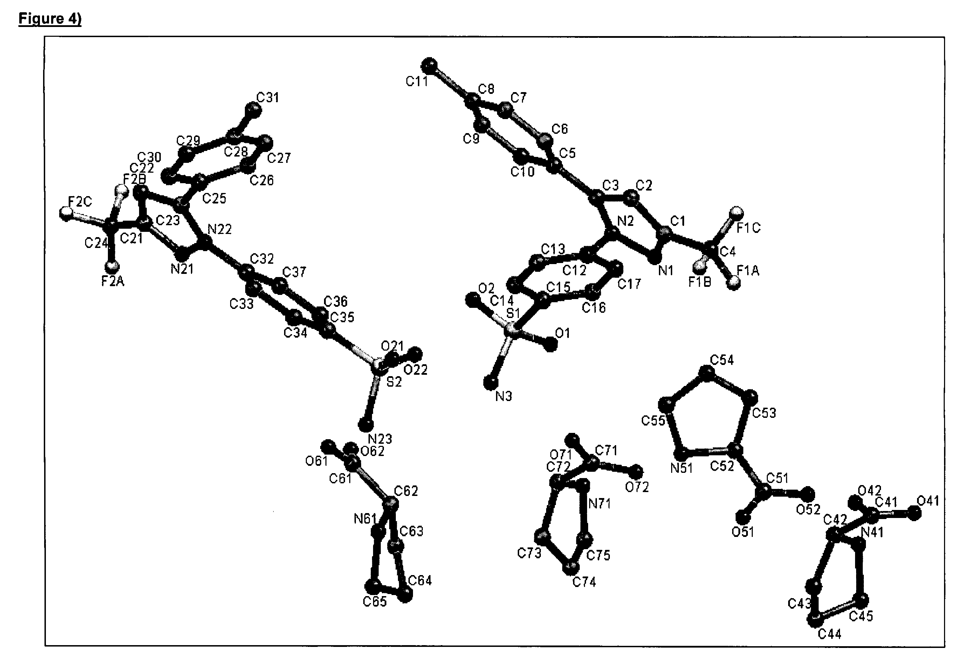

The asymmetric unit of the crystal structure of co-crystal celecoxib - L-proline (1:2) is depicted in figure 4 (hydrogen atoms have been omitted for clarity; program used: *Mercury* 2.2).

Simulation of XRPD diffractogram from single crystal data gives an almost identical diagram to the experimental one presented above.

### Example 3

### Rat model of incisional pain

### Animals

Male, Sprague-Dawley rats (Charles River Laboratories) are housed in a climate-controlled, virus free environment for at least 5 days prior to testing. Food and water are available ad *libitum* up to test time.

### Animal Dosing

The rats are all dosed intraperioneally or orally with compositions comprising a combination of tramadol hydrochloride and co-crystal celecoxib - L-proline or each agent separately, dissolved in either distilled water or dissolved in a suspension of 0.5% hydroxypropyl methylcellulose in distilled water at a fixed time. The dosing volume is 2 mL/kg. The hyperalgesic response of the animal is subsequently evaluated at a fixed later time.

### Surgery

Rats are anaesthetized with 3% isofluorane for veterinary use, employing an Ohmeda vaporizer and an anaesthesia chamber. Anaesthesia is kept during the surgical operation by a tube which directs the isofluran vapours to the animal's snout. Once the rats are anaesthetised, they are laid down in a prone position and their right hind paws are cleaned out with alcohol. Then, a 1 cm longitudinal incision is made with a number 10 scalpel, through skin and fascia of the plantar aspect of the paw, starting 0.5 cm from the proximal edge of the heel and extending toward the toes. Therefore, both superficial (skin) and deep (muscle) tissues and nerves are injured. Finally, the skin of the paw is stitched with a suturing stitch with breaded silk (3.0) and the wound is cleaned out with povidone.

### Assessment of analgesic activity against post-operative pain in rats

The drugs are tested always 4 hours after the surgery (plantar incision), 30 or 60 minutes after the administration of the product. Two behavioural endpoints are evaluated: thermal hypersensitivity or hyperalgesia, and mechanical hypersensitivity or allodynia.

### Example 3a): Assessment of thermal hypersensitivity (hyperalgesia) in post-operative pain in rats.

Hypersensitivity or hyperalgesia is assessed by measurement of a response to a thermal stimulus using a Hargreaves apparatus (Ugo Basile plantar test) which selectively elevates the temperature of an individual paw (Dirig, et al., J Neurosci Methods, 1997, 76, 183). Animals are placed in the methacrylate cages of said apparatus, having a crystal floor. The acclimation period within the cages is about 10 minutes. The thermal stimulus comes from a lamp moving below the crystal floor and which is applied to both paws, with a minimum interval of 1 minute between both stimulations in order to avoid learning behaviours. The rat is able to withdraw the paw freely when it feels the pain produced by the heat coming from the lamp; then it is switched off and the latency time to the withdrawal response is recorded in seconds. In order to avoid hurting the animal's paw, the lamp is automatically switched off after 32 seconds. Hyperalgesia is defined as a reduced latency to response compared to the latency of an untreated or vehicle treated animal, and the analgesic effect of the test compound is seen as a (partial) restoration of the latency toward normal (Dirig, et al., J Pharmcol Expt Therap, 1998, 285, 1031).

### Example 3b): Assessment of mechanical hypersensitivity (allodynia) in post-operative pain in rats.

Mechanical allodynia is tested using von Frey filaments: Animals are placed in methacrylate cylinders on an elevated surface, with metallic mesh floor perforated in order to apply the filaments. After an acclimation period of about 30 minutes within the cylinders, both hind paws are stimulated (the injured and the non-injured paw, serving the latter as control), starting with the lowest force filament (0.4 g) and reaching a 15 g filament. The animal's response to pain is manifested by the withdrawal of the paw as a consequence of the painful stimulus caused by a filament. The pressure (force in grams) threshold eliciting the withdrawal of the paw is recorded. The analgesic effect of the test compound is seen as a (partial) restoration of the threshold toward normal.

### Example 4

### Analysis of Synergistic Effect

The synergistic interaction between a tramadol hydrochloride and co-crystal celecoxib - L-proline is determined at precise dosage ratios of tramadol hydrochloride and co-crystal celecoxib - L-proline (1:2). Multiple (typically 4-6) coded doses of each selected combination are studied for effectiveness using an experimental design which permits the complete randomization of the separate dosage forms tested.

### Analysis

The analysis of possible synergistic effect for compositions at each fixed ratio is determined as disclosed by R. J. Tallarida, et al., Life Sci., 1989, 45, 947. This procedure involves the determination of the total amount in the mixture that is required to produce a specified synergistic antihyperalgesic effect at the 50% dose level (that is, the ED₅₀ or Zₘᵢₓ) and the corresponding total amount that would be expected under simple additivity (ED_{50add} or Z_{add}). Where it is established that Zₘᵢₓ <Z_{add} for a specific fixed-ratio, then that composition has a synergistic antihyperalgesic effect. Both the quantities ED₅₀ₘᵢₓ and ED_{50add} are random variables. ED₅₀ₘᵢₓ, is determined from the dose-response curve for a specific fixed-ratio of the components; ED_{50add} is calculated from the ED₅₀ values for the individual drugs. Zₘᵢₓ is then compared to Z_{add} via a Student's t- test.

## Claims

1. A compound combination of tramadol as a free base or as a pharmaceutically acceptable salt or hydrate thereof and a co-crystal of celecoxib with L-proline.

2. The compound combination according to claim 1 wherein the tramadol is (rac)-tramadol, (1*R*,1*R*)-tramadol or (1*S*,1*S*)-tramadol, preferably where the tramadol is (rac)-tramadol.

3. The compound combination according to any of claims 1 or 2 wherein the tramadol is in form of a salt, preferably in form of a hydrochloride salt; most preferably wherein the tramadol is (rac)-tramadol hydrochloride salt.

4. The compound combination according to any of claims 1 to 3 wherein the co-crystal comprises celecoxib either in neutral form or as a physiologically salt and L-proline either as a free amino acid or as a physiologically salt, preferably wherein the co-crystal comprises celecoxib in neutral form.

5. The compound combination according to any of claims 1 to 5 wherein the molecular ratio between celecoxib and L-proline in the co-crystal is 1:2.

6. The compound combination according to claim 5 wherein the endothermic sharp peak of the co-crystal corresponding to the melting point has an onset at 201.4°C.

7. The compound combination according to any of claims 5 or 6 wherein the co-crystal shows a X-Ray powder diffraction pattern with peaks [20] at 5.29, 5.77, 7.28, 9.71, 10.60, 11.57, 12.39, 13.05, 13.56, 14.46, 15.11, 15.45, 15.83, 16.14, 16.96, 17.25, 17.67, 17.92, 18.08, 18.70, 19.37, 19.64, 19.85, 20.17, 20.43, 20.79, 21.17, 21.58, 21.85, 22.15, 22.30, 22.74, 23.12, 23.78, 24.04, 24.72, 24.87, 25.43, 26.57, 26.97, 27.66, 28.04, 28.37, 28.59, 29.11, 29.56, 29.78, 30.41, 30.60, 31.56, 32.21, 32.74, 33.13, 33.35, 33.75, 34.97, 35.80, 36.87, 37.48, 37.69, 38.08 and 38.87.

8. The compound combination according to any of claims 5 to 7 wherein the co-crystal has an orthorhombic unit cell with the following dimensions:
a = 9.61 Å,
b = 20.07 Å, and
c = 30.63 Å.

9. The compound combination according to any of claims 1 to 8 wherein the ratio of the tramadol to the co-crystal celecoxib - L-proline is a weight ratio of from about 1:1 to about 1:300 and from about 1:1 to about 300:1.

10. The compound combination according to any of claims 1 to 9 wherein the ratio of the tramadol to the celecoxib is a weight ratio of from about 1:1 to about 1:30 and from about 1:1 to about 30:1.

11. A pharmaceutical composition comprising a compound combination according to any one of claims 1 to 10.

12. The pharmaceutical composition according to claim 11 for the treatment of acute to moderate pain with inflammation component.

13. Process for the production of a compound combination according to any of claims 1 to 10 comprising the steps of:
(a) dissolving or suspending celecoxib and L-proline in a solvent or mixture of solvents,
(b) optionally mixing or stirring the solution or suspension,
(c) optionally before, during or after step (b) cooling or keeping the mixed solution/suspension to or at ambient temperature or below;
(d) optionally filtering-off and/or washing the resulting solid with a solvent, and
(e) drying the solid optionally at ambient temperature and/or vacuum; followed by
(f) mixing the resulting co-crystal with tramadol as a free base or as a pharmaceutically acceptable salt or hydrate thereof.

14. Medicament comprising at least one compound combination according to any of claims 1 to 10 or pharmaceutical composition according to claim 11, and optionally one or more pharmaceutically acceptable excipients.

15. Use of a compound combination according to any one of claims 1 to 10 or pharmaceutical composition according to claim 11 in the production of a medicament for the treatment of pain, preferably acute pain, chronic pain, neuropathic pain, hyperalgesia, allodynia, cancer pain, diabetic neuropathy, diabetic peripheral neuropathy, fibromyalgia, osteoarthritis, rheumatoid arthritis, ankylosing spondylitis, sciatica, frozen shoulder or dysmenorrhea.
